# EUROPEAN PATENT SPECIFICATION

(11) **EP 2 680 696 B1**
(45) Date of publication and mention of the grant of the patent: **06.05.2015**
(21) Application number: 11845048.5
(22) Date of filing: 23.11.2011
(51) Int. Cl.: C07D 295/084, A01N 43/42

(54) **PROCESSES FOR THE PREPARATION OF ENAMINES**
VERFAHREN ZUR HERSTELLUNG VON ENAMINEN
PROCÉDÉS DE PRÉPARATION D'ÉNAMINES

(30) Priority: 03.12.2010 US 419300 P
(43) Date of publication of application: 08.01.2014
(73) Proprietor: Dow AgroSciences LLC, Indianapolis, IN 46268 (US)
(72) Inventor: BLAND, Douglas C., Midland, MI 48642 (US); TOYZAN, Todd William, Freeland, MI 48623 (US)
(74) Representative: Weickmann & Weickmann
(86) International application number: PCT/US2011/061983
(87) International publication number: WO 2012/074860

(56) References cited:
- WO-A2-2009/007460
- US-A- 3 074 940
- US-A- 3 865 791
- US-A1- 2010 004 457

## Description

### FIELD OF THE INVENTION

The invention disclosed in this document is related to the field of processes for the preparation of enamines.

### BACKGROUND OF THE INVENTION

Enamines are very useful molecules. They have been used in a wide variety of reactions such as, for example, electrophilic substitution and addition, oxidation and reduction, and cycloaddition (J. Kang, Y. R. Cho, and J. H. Lee, Bull. Korean Chem Soc. Vol. 13, No.2 , 1992).

An early method for preparing enamines involved the condensation of aldehydes and ketones with secondary amines (C. Mannich and H. Davidsen, Ber., 69, 2106 (1936). Mannich and Davidsen discovered that the condensation reaction of an aldehyde with a secondary amine could be conducted at temperatures near 0 °C in the presence of potassium carbonate (K₂CO₃), but however, the condensation reaction of a ketone with a secondary amine required calcium oxide (CaO) and elevated temperatures. Later, Herr and Heyl discovered that this type of condensation reaction could be improved by removing water (H₂O) during an azeotropic distillation with benzene (M.E. Herr and F. W. Heyl, J. Am. Chem. Soc., 74, 3627 (1952); F. W. Heyl and M.E. Herr, J. Am. Chem. Soc., 75, 1918 (1953); M.E. Herr and F. W. Heyl, J. Am. Chem. Soc., 75, 5927 (1953); F. W. Heyl and M.E. Herr, J. Am. Chem. Soc., 77, 488 (1955)). Since these publications a number of modifications have been disclosed. Usually, these modifications are based on using dehydration reagents such as K₂CO₃, CaO, *p*-toluenesulfonic acid (TsOH), boron trifluoride diethyl etherate (BF₃-OEt₂), acetic acid (AcOH), magnesium sulfate (MgSO₄), calcium hydride (CaH₂), titanium tetrachloride (TiCl₄), and molecular sieves (see J. Kang above). Other modifications deal with chemically converting water to something else during the condensation reaction (see J. Kang above). An extensive summary of the vast number of methods to prepare enamines is discussed in "ENAMINES, Synthesis, Structure, and Reactions," 2nd Edition, Edited by A. G. Cook, Chap. 2, (1988). Specific examples of processes to prepare enamines can be found in the following:
U.S. Patent 3,074,940 which discloses that certain aldehydes form azeotropes with water which can be used to remove the reaction water formed during certain enamine condensation reactions;
U.S. Patent 3,530,120 which discloses conducting certain enamine condensation reactions in an inert atmosphere with certain arsine molecules;
U.S. Patent 5,247,091 which discloses conducting certain enamine condensation reactions in an aqueous media;.
S. Kaiser, S. P. Smidt, and A. Pfaltz, Angew. Int. Ed. 2006, 45, 5194-5197 - See Supporting information pages 10-11; and
WO 2009/007460 A2, see page 13, example 1.a.
US 2010/0004457 discloses an improved process for the preparation of 2-trifluoromethyl-5-(1-substituted)alkylpyridines by cyclization, which avoids the use of the 4-alkoxy-1,1,1-trifluoro-3-buten-2-ones.
US 3,865,791 discloses enamine compounds, methods of making the same and reaction products of such enamine compounds with polyisocyanates. Said enamine compounds are prepared by the reaction of certain secondary amines and certain aldhydes and ketones.

Enamines such as 1-(3-thiobut-1-enyl)pyrrolidine are useful intermediates for the preparation of certain new insecticides (see, for example, U.S. Patent Publications 2005/0228027 and 2007/0203191). Current known processes to make such thioenamines are not efficient in producing such enamines due to a variety of reasons -- there are problems in preventing thermal degradation of the thioenamine, and while using potassium carbonate is an effective desiccant, it is problematic to filter such desiccant during larger than lab-scale production. Thus, a process is needed to remove water during these types of condensation reactions without using solid desiccants, or using temperature conditions that promote the thermal degradation of such enamines.

### DETAILED DESCRIPTION OF THE INVENTION

In general, the processes disclosed in this document can be illustrated as in Scheme 1.

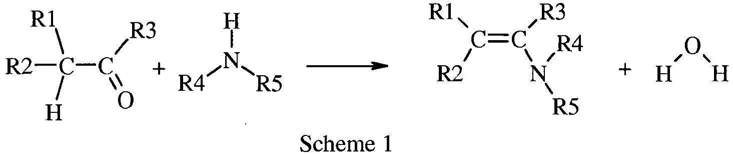

In general, the invention is a process comprising:
(A) reacting, in a reaction zone that comprises a solvent, an amine and a carbonyl to produce an enamine and H₂O
   (1) wherein said amine has the following formula 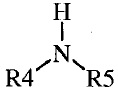 wherein R4 and R5 are each independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, or R4 and R5 taken together with N represent a 5- or 6-membered saturated or unsaturated ring; and
   (2) wherein said carbonyl (i.e. an aldehyde or a ketone) has the following formula 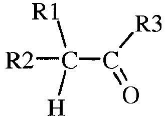
      (a) wherein R1 and R2 is each independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, each of which is independently substituted with one or more S-R6 wherein each R6 is independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, and
      (b) wherein R3 is selected from H, C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl;
   (3) wherein said reacting, in said reaction zone, is conducted under distillation conditions comprising
      (a) a pressure from about 100 Pascals (Pa) to about 120,000 Pa, and
      (b) a temperature below about, but preferably below, the thermal decomposition temperature of said enamine during said reacting; and
   (4) wherein said solvent initially comprises a non-polar-high-boiling-point liquid, a polar-high-boiling-point-liquid, and then further comprises H₂O produced from the condensation of said amine and said carbonyl to produce said enamine; and
(B) removing a vapor phase from said reaction zone wherein said vapor phase comprises H₂O.

Approximately equimolar quantities of said amine and said carbonyl can be used in the process, although excesses of one or the other may be employed. The molar ratio of amine to carbonyl can be from about 0.9 to about 1.2, however, a slight molar excess of amine to carbonyl is preferred, such as, for example, a molar ratio greater than 1 but less than about 1.1.

The reaction is conducted in the presence of a solvent that initially comprises:
(1) non-polar-high-boiling-point-liquid such as, hydrocarbon liquids, most preferably aromatic hydrocarbon liquids such as, for example, benzene, toluene, or xylene. Currently, toluene is a preferred liquid;
(2) polar-high-boiling-point-liquid such as, acetonitrile, ethanol; and then
(3) further comprises H₂O produced from the condensation of said amine and said carbonyl to produce said enamine.

In another embodiment of this invention said reacting is conducted under distillation conditions comprising a pressure from about 1000 Pa to about 60,000 Pa and a temperature from about 10 °C to about 80 °C.

In another embodiment of this invention said reacting is conducted under distillation conditions comprising a pressure from about 2500 Pa to about 30,000 Pa and a temperature from about 20 °C to about 70 °C.

In another embodiment of this invention said reacting is conducted under distillation conditions comprising a pressure from about 5000 Pa to about 15,000 Pa and a temperature from about 25 °C to about 65 °C. In another embodiment of this invention when producing 1-(3-methylsulfanyl-but-1-enyl)-pyrrolidine a temperature below about the thermal decomposition temperature of 1-(3-methylsulfanyl-but-1-enyl)-pyrrolidine during said reacting is preferred.

It is preferred in such processes that the H₂O be removed under azeotropic conditions. It is also preferred if no desiccants be used to remove H₂O.

In another embodiment of this invention, R1 and R2 are independently C₁-C₈ alkyl, C₃-C₈ cycloalkyl, each of which is independently substituted with one or more S-R6 wherein each R6 is independently selected from C₁-C₈ alkyl.

In another embodiment of this invention, R3 is H.

In another embodiment of this invention, wherein R4 and R5 are each independently selected from C₁-C₈ alkyl and C₃-C₈ cycloalkyl. In another embodiment of this invention R4 and R5 taken together with N represent a 5- or 6-membered saturated or unsaturated ring.

In another embodiment of this invention, said amine is pyrrolidine and said carbonyl is 3-methylsulfanyl-butyraldehyde. In another embodiment of this invention, said enamine is 1-(3-methylsulfanyl-but-1-enyl)-pyrrolidine.

### EXAMPLES

The examples are for illustration purposes and are not to be construed as limiting the invention disclosed in this document to only the embodiments disclosed in these examples.

### Comparative Example: Preparation of 1-(3- methylthiobut-1-enyl)pyrrolidine.

A three-neck 250 mL round bottom flask equipped with a short path distillation head was connected to a receiver flask containing a dry-ice acetone condenser. To this reaction vessel was charged 19.8 g (0.28 mol) of pyrrolidine followed by 70 mL of toluene. The mixture was cooled in an ice-water bath until the internal reaction pot temperature was about 3 °C. Then vacuum (about 3300 Pa) was applied to the system and then 94.4 g (0.14 mol) of 3-methylthiobutanal as a 17.5 wt% solution in toluene was continuously added to the reaction mixture via syringe over a one hour period. The internal reaction temperature rose from 3 °C up to 18 °C during addition of the aldehyde solution. Distillate was also collected during aldehyde addition. Upon completing addition of the 3-methylthiobutanal solution, the distillation was continued for an additional 50 minutes (min) until the internal pot temperature reached 26 °C. At this time, the vacuum was adjusted to about 2400 Pa and the distillation was continued for an additional 2.0 min until the internal pot temperature reached 24 °C. The distillation was stopped and the reaction vessel was padded with nitrogen. The reactive distillation bottoms were isolated to give 74.91 g of 1-(3-methylthiobut-1-enyl)pyrrolidine was a 28 wt% yellow solution in toluene. Proton (¹H) NMR spectroscopic assay of the solution mixture (using benzyl acetate as the internal standard) indicated a 84% in-pot yield.

### Example 1: Preparation of 1-(3-methylthiobut-1-enyl)pyrrolidine.

To a 3-Liter three-neck round bottom flask equipped with mechanical stirring, short path distillation head, and nitrogen padding was charged with 61 g (0.86 mol) of pyrrolidine followed by 100 mL of toluene and 200 mL of acetonitrile (33% toluene in acetonitrile v/v). The mixture was cooled in an ice-water bath and then 558 g (0.78 mol) of a 16.5 wt% 3-methylthiobutanal in toluene solution was continuously added via additional funnel over a 130 min period. The internal reaction temperature was maintained below 7 °C during the aldehyde addition. The ice-water bath was removed and a pressure of about 6600 Pa was applied to the system. The reaction mixture was heated up to 15 °C (pot temperature) at which time distillate began to be collected overhead. The internal reaction temperature was heated until the pot temperature reached 33 °C. Total time for the distillation was about 1 h. The reaction mixture was padded with nitrogen and then cooled down to ambient temperature. A total of 282.4 g of overhead distillate was collected. The reaction distillation bottoms were collected to give a about 25.0 wt% 1-(3-methylthiobut-1-enyl)pyrrolidine in toluene solution (yield was approximated to be 89% based on ¹H NMR spectroscopy using benzyl acetate as an internal standard).

## Claims

1. A process comprising:
(A) reacting, in a reaction zone that comprises a solvent, an amine and a carbonyl to produce an enamine and H₂O 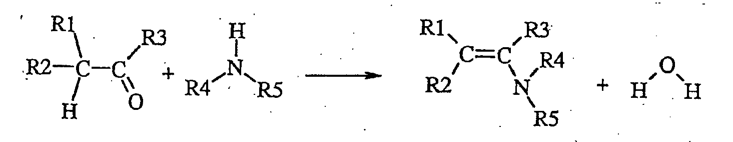
(1) wherein said amine has the following formula 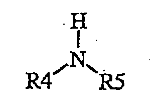 wherein R4 and R5 are each independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, or R4 and R5 taken together with N represent a 5- or 6-membered saturated or unsaturated ring; and
(2) wherein said carbonyl (i.e. an aldehyde or a ketone) has the following formula 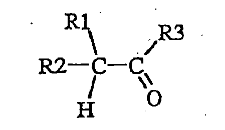
(a) wherein R1 and R2 is each independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, each of which is independently substituted with one or more S-R6 wherein each R6 is independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, and
(b) wherein R3 is selected from H, C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl;
or said carbonyl is 3-methylsulfanyl-butyraldehyde;
(3) wherein said reacting, in said reaction zone, is conducted under distillation conditions comprising
(a) a pressure from about 100 Pascals (Pa) to about 120,000 Pa, and
(b) a temperature below about, but preferably below, the thermal decomposition temperature of said enamine during said reacting; and
(4) wherein said solvent initially comprises a non-polar-high-boiling-point liquid, a polar-high-boiling-point-liquid, and then further comprises H₂O produced from the condensation of said amine and said carbonyl to produce said enamine; and
(B) removing a vapor phase from said reaction zone wherein said vapor phase comprises H₂O.

2. A process according to claim 1 wherein approximately equimolar quantities of said amine and said carbonyl can be used in the process.

3. A process according to claim 1 wherein the molar ratio of amine to carbonyl is from about 0.9 to about 1.2 or wherein molar ratio of amine to carbonyl is greater than 1 but less than about 1.1.

4. A process according to claim 1 wherein the reaction is conducted in the presence of a solvent that initially comprises said non-polar-high-boiling-point-liquid where said liquid is benzene or
wherein the reaction is conducted in the presence of a solvent that initially comprises said non-polar-high-boiling-point-liquid where said liquid is toluene or
wherein the reaction is conducted in the presence of a solvent that initially comprises said non-polar-high-boiling-point-liquid where said liquid is xylene or
wherein the reaction is conducted in the presence of a solvent that initially comprises said polar-high-boiling-point-liquid where said liquid is acetonitrile or wherein the reaction is conducted in the presence of a solvent that initially comprises said polar-high-boiling-point-liquid where said liquid is ethanol.

5. A process according to claim 1 wherein said reacting is conducted under distillation conditions comprising a pressure from about 1000 Pa to about 60,000 Pa and a temperature from about 10 °C to about 80 °C or wherein said reacting is conducted under distillation conditions comprising a pressure from about 2500 Pa to about 30,000 Pa and a temperature from about 20 °C to about 70 °C or wherein said reacting is conducted under distillation conditions comprising a pressure from about 5000 Pa to about 15,000 Pa and a temperature from about 25 °C to about 65 °C.

6. A process according to claim 1 wherein 1-(3-methylsulfanyl-but-1-enyl)-pyrrolidine is produced at a temperature below about the thermal decomposition temperature of 1-(3- methylsulfanyl-but-1-enyl)-pyrrolidine.

7. A process according to claim 1 wherein said H₂0 is removed under azeotropic conditions.

8. A process according claim 1 wherein no desiccants are used to remove H₂0.

9. A process according to claim 1 wherein said R1 and R2 are independently C₁-C₈ alkyl, C₃-C₈ cycloalkyl, each of which is independently substituted with one or more S-R6 wherein each R6 is independently selected from C₁-C₈ alkyl.

10. A process according to claim 1 wherein R3 is H.

11. A process according to claim 1 wherein R4 and R5 are each independently selected from C₁-C₈ alkyl and C₃-C₈ cycloalkyl.

12. A process according to claim 1 wherein R4 and R5 taken together with N represent a 5- or 6-membered saturated or unsaturated ring.

13. A process according to claim 1 wherein said amine is pyrrolidine and said carbonyl is 3-methylsulfanyl-butyraldehyde.

14. A process according to claim 1 wherein said enamine is 1-(3-methylsulfanyl-but-1-enyl)-pyrrolidine.

15. A process according to claim 1 comprising:
(A) reacting, in a reaction zone that comprises a solvent, pyrrolidine and 3-methylsulfanyl-butyraldehyde to produce 1-(3-methylsulfanyl-but-1-enyl)-pyrrolidine and H₂0, wherein said reacting, in said reaction zone, is conducted under distillation conditions comprising
(1) a pressure from about 5000 Pascals (Pa) to about 15,000 Pa, and
(2) a temperature from about 25 °C to about 65 °C; and
wherein said solvent initially comprises toluene and acetonitrile, and then further comprises H₂0 produced from the condensation of said pyrrolidine and said 3-methylsulfanyl-butyraldehyde to produce said 1-(3-methylsulfanyl-but-1-enyl)-pyrrolidine; and
(B) removing a vapor phase from said reaction zone wherein said vapor phase comprises H₂0.

## Patentansprüche

1. Verfahren, umfassend:
(A) Umsetzen in einer Reaktionszone, die ein Lösungsmittel umfasst, eines Amins und eines Carbonyl, um ein Enamin und H₂O zu bilden 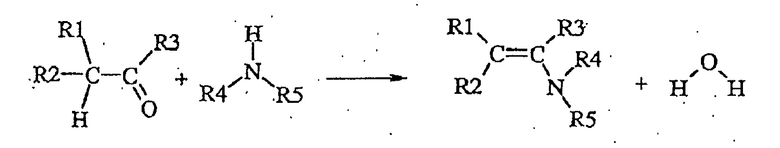
(1) worin das Amin die folgende Formel 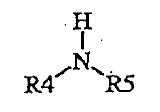 aufweist,
worin R4 und R5 unabhängig ausgewählt werden aus C₁-C₈-Alkyl, C₃-C₈-Cycloalkyl, C₂-C₈-Alkoxyalkyl, C₇-C₁₂-Arylalkyl, C₂-C₈-Alkylaminoalkyl, Aryl und Heteroaryl oder R4 und R5 zusammengenommen mit N einen 5- oder 6-gliedrigen gesättigten oder ungesättigten Ring bedeuten; und
(2) worin das Carbonyl (d.h. ein Aldehyd oder Keton) die folgende Formel aufweist: 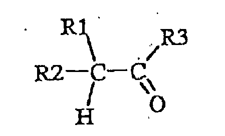
(a) worin R1 und R2 jeweils unabhängig ausgewählt werden aus C₁-C₈- Alkyl, C₃-C₈-Cycloalkyl, C₂-C₈-Alkoxyalkyl, C₇-C₁₂-Arylalkyl, C₂-C₈-Alkylaminoalkyl, Aryl und Heteroaryl, wobei jedes davon unabhängig substituiert ist mit einem oder mehreren S-R6, worin jedes R6 unabhängig ausgewählt ist aus C₁-C₈-Alkyl, C₃-C₈-Cycloalkyl, C₂-C₈-Alkoxyalkyl, C₇-C₁₂-Arylalkyl, C₂-C₈-Alkylaminoalkyl, Aryl und Heteroaryl, und
(b) worin R3 ausgewählt wird aus H, C₁-C₈-Alkyl, C₃-C₈-Cycloalkyl, C₂-C₈-Alkoxyalkyl, C₇-C₁₂-Arylalkyl, C₂-C₈-Alkylaminoalkyl, Aryl und Heteroaryl;
oder das Carbonyl 3-Methylsulfanyl-butyraldehyd ist;
(3) worin das Umsetzen in der Reaktionszone unter Destillationsbedingungen durchgeführt wird, umfassend
(a) einen Druck von etwa 100 Pascal (Pa) bis etwa 120000 Pa und
(b) eine Temperatur unter etwa, aber vorzugsweise unter, der thermischen Zersetzungstemperatur des Enamins während des Umsetzens; und
(4) worin das Lösungsmittel anfangs eine unpolare hochsiedende Flüssigkeit, eine polare hochsiedende Flüssigkeit und dann weiterhin H₂O umfasst, das durch die Kondensationsreaktion des Amins und des Carbonyls zur Bildung des Enamins gebildet wird; und
(B) Entfernen einer Dampfphase aus der Reaktionszone, worin die Dampfphase H₂O umfasst.

2. Verfahren nach Anspruch 1, worin ungefähr äquimolare Mengen des Amins und des Carbonyls in dem Verfahren verwendet werden können.

3. Verfahren nach Anspruch 1, worin das molare Verhältnis von Amin zu Carbonyl von etwa 0,9 bis etwa 1,2 ist oder worin das molare Verhältnis von Amin zu Carbonyl größer als 1 aber kleiner als etwa 1,1 ist.

4. Verfahren nach Anspruch 1, worin die Reaktion in der Gegenwart eines Lösungsmittels durchgeführt wird, das anfangs unpolare hochsiedende Flüssigkeit umfasst, wobei die Flüssigkeit Benzol ist, oder
worin die Reaktion in der Gegenwart eines Lösungsmittels durchgeführt wird, das anfangs die unpolare hochsiedende Flüssigkeit umfasst, wobei die Flüssigkeit Toluol ist; oder
worin die Reaktion in der Gegenwart eines Lösungsmittels durchgeführt wird, das anfangs die unpolare hochsiedende Flüssigkeit umfasst, wobei die Flüssigkeit Xylol ist; oder
worin die Reaktion in der Gegenwart eines Lösungsmittels durchgeführt wird, das anfangs die polare hochsiedende Flüssigkeit umfasst, wobei die Flüssigkeit Acetonitril ist; öder
worin die Reaktion in der Gegenwart eines Lösungsmittels durchgeführt wird, das anfangs die polare hochsiedende Flüssigkeit umfasst, wobei die Flüssigkeit Ethanol ist.

5. Verfahren nach Anspruch 1, worin die Umsetzung unter Destillationsbedingungen durchgeführt wird, umfassend einen Druck von etwa 1000 Pa bis etwa 60000 Pa und eine Temperatur von etwa 10 °C bis etwa 80 °C, oder worin die Umsetzung unter Destillationsbedingungen durchgeführt wird, umfassend einen Druck von etwa 2500 Pa bis etwa 30000 Pa und eine Temperatur von etwa 20 °C bis etwa 70 °C, oder worin die Umsetzung unter Destillationsbedingungen durchgeführt wird, umfassend einen Druck von etwa 5000 Pa bis etwa 15000 Pa und eine Temperatur von etwa 25 °C bis etwa 65 °C.

6. Verfahren nach Anspruch 1, worin 1-(3-Methylsulfanyl-but-1-enyl)-pyrrolidin bei einer Temperatur unter etwa der thermischen Zersetzungstemperatur von 1-(3-Methylsulfanyl-but-1-enyl)-pyrrolidin gebildet wird.

7. Verfahren nach Anspruch 1, worin das H₂O unter azeotropen Bedingungen entfernt wird.

8. Verfahren nach Anspruch 1, worin keine Trocknungsmittel zum Entfernen von H₂O verwendet werden.

9. Verfahren nach Anspruch 1, worin R1 und R2 unabhängig C₁-C₈-Alkyl, C₃-C₈-Cycloalkyl sind, wobei jedes davon unabhängig substituiert ist mit einem oder mehreren S-R6, worin jedes R6 unabhängig ausgewählt wird aus C₁-C₈-Alkyl.

10. Verfahren nach Anspruch 1, worin R3 H ist.

11. Verfahren nach Anspruch 1, worin R4 und R5 jeweils unabhängig ausgewählt werden aus C₁-C₈-Alkyl und C₃-C₈-Cycloalkyl.

12. Verfahren nach Anspruch 1, worin R4 und R5 zusammengenommen mit N einen 5- oder 6-gliedrigen gesättigten oder ungesättigten Ring bedeuten.

13. Verfahren nach Anspruch 1, worin das Amin Pyrrolidin ist und das Carbonyl 3-Methylsulfanyl-butyraldehyd ist.

14. Verfahren nach Anspruch 1, worin das Enamin 1-(3-Methylsulfanyl-but-1-enyl)-pyrrolidin ist.

15. Verfahren nach Anspruch 1, umfassend:
(A) Umsetzen in einer Reaktionszone, die ein Lösungsmittel umfasst, von Pyrrolidin und 3-Methylsulfanyl-butyraldehyd, um 1-(3-Methylsulfanyl-but-1-enyl)-pyrrolidin und H₂O zu bilden
worin das Umsetzen in der Reaktionszone unter Destillationsbedingungen durchgeführt wird, umfassend:
(1) einen Druck von etwa 5000 Pascal (Pa) bis etwa 15000 Pa
und
(2) eine Temperatur von etwa 25 °C bis etwa 65 °C; und
worin das Lösungsmittel anfangs Toluol und Acetonitril und dann weiterhin H₂O umfasst, das gebildet wird durch die Kondensationsreaktion des Pyrrolidins und des 3-Methylsulfanyl-butyraldehyds zur Bildung des 1-(3-Methylsulfanyl-but-1-enyl)-pyrrolidins; und
(B) Entfernen einer Dampfphase aus der Reaktionszone, worin die Dampfphase H₂O umfasst.

## Revendications

1. Procédé comprenant les étapes suivantes :
A) faire réagir, dans une zone de réaction qui contient un solvant, une amine et un composé carbonylé, pour produire une énamine et de l'eau : 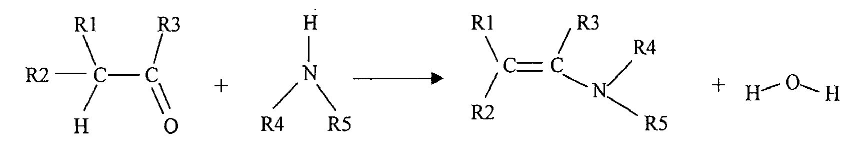 étant entendu
1) que ladite amine a la formule suivante : 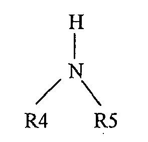 dans laquelle R4 et R5 représentent chacun, indépendamment, une entité choisie parmi les groupes alkyle en C₁-C₈, cycloalkyle en C₃-C₈, alcoxy-alkyle en C₂-C₈, aryl-alkyle en C₇-C₁₂, alkyl-amino-alkyle en C₂-C₈, aryle et hétéroaryle, ou bien R4 et R5 représentent, conjointement avec N, un cycle saturé ou insaturé, comportant 5 ou 6 chaînons,
2) que ledit composé carbonylé, c'est-à-dire aldéhyde ou cétone, a la formule suivante : 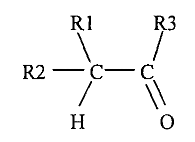 dans laquelle
a) R1 et R2 représentent chacun, indépendamment, une entité choisie parmi les groupes alkyle en C₁-C₈, cycloalkyle en C₃-C₈, alcoxy-alkyle en C₂-C₈, aryl-alkyle en C₇-C₁₂, alkyl-amino-alkyle en C₂-C₈, aryle et hétéroaryle, qui portent chacun, indépendamment, un ou plusieurs substituant(s) de formule -S-R6 où chaque symbole R6 représente une entité choisie parmi les groupes alkyle en C₁-C₈, cycloalkyle en C₃-C₈, alcoxy-alkyle en C₂-C₈, aryl-alkyle en C₇-C₁₂, alkyl-amino-alkyle en C₂-C₈, aryle et hétéroaryle,
b) et R3 représente une entité choisie parmi un atome d'hydrogène et les groupes alkyle en C₁-C₈, cycloalkyle en C₃-C₈, alcoxy-alkyle en C₂-C₈, aryl-alkyle en C₇-C₁₂, alkyl-amino-alkyle en C₂-C₈, aryle et hétéroaryle,
ou que ledit composé carbonylé est du 3-(méthyl-sulfanyl)-butyraldéhyde ;
3) que ladite réaction, dans ladite zone de réaction, est menée dans des conditions de distillation comprenant :
a) une pression qui vaut d'environ 100 Pa (pascals) à environ 120 000 Pa,
b) et une température qui est inférieure à environ la température de décomposition thermique de ladite énamine au cours de ladite réaction, et de préférence, inférieure à cette température ;
4) et que ledit solvant comprend initialement un liquide non-polaire à haut point d'ébullition, un liquide polaire à haut point d'ébullition, et comprend en outre de l'eau, qui est générée par la condensation de ladite amine et dudit composé carbonylé qui donne ladite énamine ;
B) et chasser une phase vapeur de ladite zone de réaction, laquelle phase vapeur comprend de l'eau.

2. Procédé conforme à la revendication 1, dans lequel procédé on peut utiliser des quantités à peu près équimolaires de ladite amine et dudit composé carbonylé.

3. Procédé conforme à la revendication 1, dans lequel le rapport molaire de l'amine au composé carbonylé vaut d'environ 0,9 à environ 1,2, ou dans lequel le rapport molaire de l'amine au composé carbonylé est supérieur à 1, mais inférieur à environ 1,1.

4. Procédé conforme à la revendication 1,
- dans lequel la réaction est menée en présence d'un solvant qui comprend initialement dudit liquide non-polaire à haut point d'ébullition, lequel liquide est du benzène ;
- ou dans lequel la réaction est menée en présence d'un solvant qui comprend initialement dudit liquide non-polaire à haut point d'ébullition, lequel liquide est du toluène ;
- ou dans lequel la réaction est menée en présence d'un solvant qui comprend initialement dudit liquide non-polaire à haut point d'ébullition, lequel liquide est du xylène ;
- ou dans lequel la réaction est menée en présence d'un solvant qui comprend initialement dudit liquide polaire à haut point d'ébullition, lequel liquide est de l'acétonitrile ;
- ou dans lequel la réaction est menée en présence d'un solvant qui comprend initialement dudit liquide polaire à haut point d'ébullition, lequel liquide est de l'éthanol.

5. Procédé conforme à la revendication 1,
- dans lequel ladite réaction est menée dans des conditions de distillation comprenant une pression d'environ 1 000 Pa à environ 60 000 Pa et une température d'environ 10 °C à environ 80 °C ;
- ou dans lequel ladite réaction est menée dans des conditions de distillation comprenant une pression d'environ 2 500 Pa à environ 30 000 Pa et une température d'environ 20 °C à environ 70 °C ;
- ou dans lequel ladite réaction est menée dans des conditions de distillation comprenant une pression d'environ 5 000 Pa à environ 15 000 Pa et une température d'environ 25 °C à environ 65 °C.

6. Procédé conforme à la revendication 1, dans lequel on produit de la 1-[3-(méthyl-sulfanyl)-but-1-ényl]-pyrrolidine, à une température inférieure à environ la température de décomposition thermique de la 1-[3-(méthyl-sulfanyl)-but-1-ényl]-pyrrolidine.

7. Procédé conforme à la revendication 1, dans lequel ladite eau est chassée dans des conditions azéotropiques.

8. Procédé conforme à la revendication 1, dans lequel on n'utilise pas d'agents de dessiccation pour chasser l'eau.

9. Procédé conforme à la revendication 1, dans lequel lesdits symboles R1 et R2 représentent chacun, indépendamment, un groupe alkyle en C₁-C₈ ou cycloalkyle en C₃-C₈, qui porte dans chaque cas, indépendamment, un ou plusieurs substituant(s) de formule -S-R6 où chaque symbole R6 représente indépendamment une entité choisie parmi les groupes alkyle en C₁-C₈.

10. Procédé conforme à la revendication 1, dans lequel R3 représente un atome d'hydrogène.

11. Procédé conforme à la revendication 1, dans lequel R4 et R5 représentent chacun, indépendamment, une entité choisie parmi les groupes alkyle en C₁-C₈ et cycloalkyle en C₃-C₈.

12. Procédé conforme à la revendication 1, dans lequel R4 et R5 représentent, conjointement avec N, un cycle saturé ou insaturé, comportant 5 ou 6 chaînons.

13. Procédé conforme à la revendication 1, dans lequel ladite amine est de la pyrrolidine et ledit composé carbonylé est du 3-(méthyl-sulfanyl)-butyraldéhyde.

14. Procédé conforme à la revendication 1, dans lequel ladite énamine est de la 1-[3-(méthyl-sulfanyl)-but-1-ényl]-pyrrolidine.

15. Procédé conforme à la revendication 1, comportant les étapes suivantes :
A) faire réagir, dans une zone de réaction qui contient un solvant, de la pyrrolidine et du 3-(méthyl-sulfanyl)-butyraldéhyde, pour produire de la 1-[3-(méthyl-sulfanyl)-but-1-ényl]-pyrrolidine et de l'eau, étant entendu que ladite réaction, dans ladite zone de réaction, est menée dans des conditions de distillation comprenant :
1) une pression d'environ 5 000 Pa (pascals) à environ 15 000 Pa,
2) et une température d'environ 25 °C à environ 65 °C,
et que ledit solvant comprend initialement du toluène et de l'acétonitrile, et comprend en outre de l'eau, qui est générée par la condensation de ladite pyrrolidine et dudit 3-(méthyl-sulfanyl)-butyraldéhyde, qui donne de ladite 1-[3-(méthyl-sulfanyl)-but-1-ényl]-pyrrolidine ;
B) et chasser une phase vapeur de ladite zone de réaction, laquelle phase vapeur comprend de l'eau.
